# EUROPEAN PATENT APPLICATION

(11) **EP 2 769 734 A1**
(43) Date of publication of application: **27.08.2014**
(21) Application number: 14166316.1
(22) Date of filing: 05.08.2010
(51) Int. Cl.: A61K 39/05, A61K 39/39, A61K 35/74, A61K 39/00

(54) **A vaccine directed against porcine pleuropneumonia and a method to obtain such a vaccine**

(30) Priority: 06.08.2009 EP 09167342; 06.08.2009 US 231820 P
(62) Divisional of application: 10739632.7
(71) Applicant: Intervet International B.V., 5831 AN Boxmeer (NL)
(72) Inventor: Klaasen, Henricus Leo Bernardus Maria, 5831 AN Boxmeer (NL)
(74) Representative: Janssen, Paulus J. P.

(57) **Abstract**

The present invention pertains to a vaccine directed against porcine pleuropneumonia, comprising lipopolysaccharide, wherein the vaccine comprises a polymyxin to reduce symptoms of an endotoxic shock arising from the lipopolysaccharide. The invention also pertains to a method to obtain such a vaccine and a method for administering the vaccine to a subject animal.

## Description

The present invention pertains to a vaccine directed against porcine pleuropneumonia, a world-wide disease causing substantial economic loss to the swine industry. The etiological agent of porcine pleuropneumonia is *Actinobacillus pleuropneumoniae* (APP), a gram-negative bacterium belonging to the family *Pasteurellaceae.* The disease is transmitted by the aerosol route or direct contact with an infected pig, and is characterized by hemorrhagic, fibrinous and necrotic lung lesions. The clinical picture may range from peracute to chronic and asymptomatic carrier pigs can transmit the disease when introduced into uninfected herds. Based on capsular polysaccharides and lipopolysaccharide (LPS) O-chain components, 15 serovars have been described. Serotyping and other genetic typing methods for *Actinobacillus pleuropneumoniae* have contributed greatly to surveillance and epidemiological studies. These tools provide important information for decision making in control programs aimed at eradication of virulent types of the bacterium. In North America, serovars 1, 5 and 7 are reported to be the most prevalent, while serovars 2 and 9 are most commonly isolated in Europe, and serovar 15 is the predominant isolate from Australian pigs.

The virulence factors described for *A*. *pleuropneumoniae* include LPS, capsular polysaccharides, Apx toxins I-IV (so called repeats in toxins or RTX toxins), outer membrane proteins (OMPs) and various iron acquisition systems. However, the overall contribution of each component to the infection process remains unclear, as do the mechanisms of pathogenesis of this bacterium. Almost all of the currently available vaccines against *A. pleuropneumoniae* are either inactivated whole-cell bacterins or subunit combinations of Apx toxins and proteins, optionally in combination with outer membrane fractions. In any case, it has become commonly understood that for obtaining adequate protection, preferably across different serovars, lipopolysaccharide (LPS) has to be present in a vaccine (Julien Gouré et al. in: BMC Genomics 2009, 10:88, published 24 February 2009; Dubreuil et al. in: Animal Health Research Reviews 1(2): 73-93, 2000 December). Even stronger, it has been known since the 1980's that a vaccine based on lipopolysaccharide alone already has a positive effect in reducing the level of infection or the clinical signs of disease and induce cross-protection (Fenwick et al. in: the American Journal of Veterinary Research, Volume 47, No 9, September 1986; Rapp et al. in: Canadian Veterinary Journal, 1988, Volume 29, Number 7, pp 585-587; Rioux et al. in: Comparative Immunology, Microbiology & Infectious Diseases 20(1):63-74, January 1997).

A serious disadvantage of lipopolysaccharide is its endotoxic nature, thus being associated with multiple unwanted side effects such as lethargy, diarrhoea, vomiting, loss of appetite or even death. At first glance it is therefore tempting to lower the amount of LPS in a vaccine or to detoxify the LPS. However, an adequate APP vaccine depends on the presence of LPS and thus, as is commonly known, lowering the amount of LPS inherently decreases the efficacy of the vaccine. Also, various attempts have been made to reduce the endotoxic nature of LPS and still arrive at an effective APP vaccine. Rioux et al. (mentioned here-above) investigated the effect of detoxifying LPS on the efficacy of a vaccine against APP and concluded that mice immunized with (non detoxified) LPS were protected, whereas a vaccine based on detoxified LPS failed to protect the mice (see page 71 of the Rioux publication, third paragraph). This study was repeated in pigs (Research in Veterinary Science 1998, 65, 165-167). It was shown that a commercial bacterin vaccine (containing non detoxified LPS) provided good protection against a challenge with wild type APP. A vaccine based on detoxified LPS appeared to be far less effective (60% of the pigs still had lung lesions). Indeed, this is in line with the common understanding that LPS, when detoxified, is far less immunogenic.

Object of the present invention is to provide a vaccine directed against porcine pleuropneumonia, having an efficacy comparable with commercially obtainable vaccines, but having significantly reduced symptoms of endotoxic shock, in particular showing no or hardly any signs of diarrhoea, vomiting and increased respiration in pigs after administration of an effective dosis of the vaccine.

To this end, a vaccine has been devised based comprising lipopolysaccharide, wherein the vaccine additionally comprises a polymyxin to reduce symptoms of an endotoxic shock arising from the lipopolysaccharide. Surprisingly it has been found that the detoxification of LPS by a polymyxin does not have a significant negative effect on the immunogenicity of the LPS and thus on the efficacy of the vaccine. It appears that by using a polymyxin symptoms of an endotoxic shock, such as diarrhoea, vomiting and increased respiration can be significantly reduced while maintaining the efficacy of the vaccine at an adequate level. In particular, it appears that the vaccine may even have the same efficacy as a vaccine without added polymyxin.

A vaccine in the sense of the present invention is a constitution that aims at preventing or reducing an infection by a micro-organism or the clinical symptoms of the disease or disorder caused by that infection through induction of antibodies that interfere with the micro-organism or its metabolic processes (either in, or induced by the micro-organism) in the host. A vaccine may contain a sub-unit of the micro-organism (such as for example LPS) as the basic antigen, but may also comprise attenuated or killed whole microorganisms, or for example a metabolite of the organism (such as an excreted toxin). The vaccine contains an immunologically effective amount of the antigen (i.e. an amount capable of stimulating the immune system of the target animal sufficiently to at least reduce the negative effects of a challenge with wild-type micro-organisms), typically combined with a pharmaceutically acceptable carrier such as a liquid containing water (often phosphate buffered saline), optionally comprising immunostimulating agents (adjuvants), stabilisers, viscosity modifiers or other components depending on the intended use or required properties of the vaccine. Also, additional antigens of other micro-organisms may be present such that the vaccine protects against multiple wild-type organisms.

Polymyxins are compounds with antibacterial properties which can be isolated from *Bacillus polymyxa* (*B. aerospora*), or artificially produced, and compounds with antibacterial properties directly derivable therefrom. The polymyxins produced by *B. polymyxa* have a general structure consisting of a cyclic peptide with a long hydrophobic tail. Typical polymyxins are: polymyxin A (also called aerosporin), polymyxin B (often also referred to as polymyxin), polymyxin E (also called colistin), polymyxin M (also known as mattacin) and polymyxin nonapeptide.

It is noted that it has been described before that polymyxins can be used to successfully detoxify lipopolysaccharides in immunogenic compositions containing gram-negative bacteria. For example, Cooperstock et al disclose in the journal "Infection and Immunity", July 1981, pp 315-318 that by using polymyxin B, endotoxic activity in suspensions of several gram-negative bacteria (viz. *Bordetella pertussis, Escherichia coli, Haemophilus influenzae* and *Pseudomonas aeruginosa*) was markedly decreased. However, Cooperstock also clearly notes that "it remains uncertain whether the immunogenic properties of polymyxin-treated bacteria remain intact" and also that "the ability of several ...outer membrane components to bind preformed antibody in an immunoelectrophoresis system was partially lost after treatment of either whole bacteria or isolated membranes with high concentrations of Polymyxin B". In other words: the immunogenic properties of the detoxified LPS were seriously questioned, and loss of at least part of these properties was foreseen.

Indeed, also Bannatyne et al. disclose in the Journal of Hygiene (Cambridge), 1981, 87, pp 377-381, that polymyxin in a concentration of 5000 µg (polymyxine B sulphate) per ml (which equals about 42.000 International Units per dose of the vaccine) were successful in suppressing the toxic effect of LPS. However, he also states "There remain, however, reservations about such a polymyxin-containing vaccine. Most importantly is whether reduction in endotoxin content is accompanied by a reduction in the amount of protective antigen and associated with a drop in protective efficacy." A few years later Larter et al. (in the American Journal of Diseases of Children, Vol 138, March 1984) indeed found that in a pertussis vaccine, the antibody titres in animals receiving pertussis vaccine and polymyxin concomitantly (over 150.000 IU per dose) were lower and rose more slowly or were even not present at all.

These findings in the early 1980's might explain why for an effective APP vaccine, in which vaccine LPS plays a dominant role, detoxification with a polymyxin was never believed to have a reasonable chance of success to obtain a safe but still effective vaccine. This is strongly supported by the fact that over 25 years have passed since the observations mentioned here-above, until the present application of a polymyxin in an APP vaccine comprising LPS as an essential constituent.

In an embodiment the vaccine comprises lipopolysaccharide purified from a bacterial culture (i.e. being concentrated relative to other cell components such as cell organelles, the nucleus, cell wall components etc.) complexed with one or more repeats in toxins. It is known that an APP vaccine based on bacterins offers less protection than a vaccine based on a combination of LPS- and Apx subunits in the form of a complex. Therefore such a combination subunit vaccine, comprising LPS purified from a bacterial culture complexed with one or more Apx toxins is preferred. It is known that the specific binding between LPS and these toxins plays a major role in the improved immunogenicity of the toxins (see i.a. Ramjeet et al. in Molecular Biology, 2008, 70(1), pp 221-235) which may explain the improved protection of such a combination sub-unit vaccine. It was surprisingly found now that polymyxin, despite its strong LPS binding properties, did not seem to negatively interfere with this LPS-toxin complex, since a combination sub-unit vaccine comprising polymyxin offers protection equal to the same combination sub-unit vaccine not comprising a polymyxin. The repeats in toxins may for example be the ones known from EP 453 024. Also, any of the other repeats in toxins ApxII or ApxIV may be present. This may depend i.a. on the APP serovar(s) one wants to protect against. It is known that next to *Actinobacillus pleuropneumoniae,* Apx toxins (I and II) are also produced by *Actinobacillus suis.* The latter toxins may also be used in the vaccine according to this embodiment. Preferably one or more of the toxins Apx I, Apx II and Apx III are comprised in the vaccine, more preferably each of these.

In an embodiment the vaccine comprises less than 2000 IU of the polymyxin per dose. It has surprisingly been found that an effective reduction of the endotoxic symptoms can be arrived at by using as little as 2000 IU/dose (which can be arrived at by for example adding about 60 µg polymyxine B sulphate per ml vaccine for a 4 ml dose). In the references cited here-above, effective amounts when vaccinating lie in the range of 42.000 - 160.0000 IU polymyxin per dose, which is about the range for the use of polymyxin as a preservative (see also 9^{th} edition of the Merck Veterinary Manual, ed. 2005, which recommends a dose of at least 50.000 IU per dose for a 10 kg animal). Applicant surprisingly found that only a fraction (less than 5 %) appears to be sufficient to reduce symptoms of an endotoxic shock. Since polymyxins are generally not indefinitively stable, the amount present in the vaccine will be less than the amount added, typically between 50 and 80% when measured up to one year after formulation. It is noted that as little polymyxin as necessary to reduce clinical symptoms can be used. It may for example be that in a breed of swine which suffers less from LPS in a vaccine, an amount of about 15-30 IU per dose is effective in adequately reducing symptoms of an endotoxic shock. The positive effect of such a low amount has been confirmed experimentally. It is noted that minimum effective amount may not only depend on the breed of the pigs, but also on the reduction of clinical symptoms required, the amount of LPS in the vaccine, the type of polymyxin etc. In an embodiment, the vaccine comprises less than 1000, or even less than 500 IU per dose.

In an embodiment the lipopolysaccharide originates from *Actinobacillus pleuropneumoniae.* Indeed, as is commonly known, the LPS in the vaccine need not be extracted from APP bacteria to be effective in a vaccine against porcine pleuropneumonia. It may originate for example from *Escherichia coli,* be recombinantly expressed in a host bacterium, animal cell or other type of host. However, we found that LPS that originates from APP gives very good vaccination results. Such LPS may be extracted directly from APP bacteria but may also be expressed by a suitable host.

In an embodiment, the vaccine comprises a 42 kD outer membrane protein of *Actinobacillus pleuropneumoniae* as known from EP 453 024.

The present invention also pertains to a method to obtain a vaccine directed against porcine pleuropneumonia, comprising obtaining lipopolysaccharide, mixing the lipopolysaccharide with a pharmaceutically acceptable carrier and detoxifying the lipopolysaccharide by adding a polymyxin. Detoxifying in this sense means that an amount of polymyxin is added suitable to at least reduce symptoms of an endotoxic shock, in particular diarrhoea, vomiting and increased respiration. Preferably less than 2000, or even less than 1000 or 500 IU of polymyxin are used per intended dosis of the vaccine. In an embodiment the polymyxin is added when the lipopolysaccharide is mixed with the carrier.

The invention also pertains to a method to reduce symptoms of an endotoxic shock when administering a vaccine directed against porcine pleuropneumonia containing lipopolysaccharide, comprising administering a vaccine according to the present invention.

The invention will now be illustrated using the following examples.

### Experimental design

In a first study 150 specific pathogen-free (SPF) pigs aged 6 weeks were used, and three groups of 50 animals each were formed. Each group was vaccinated once intramuscularly with a 4 ml dose at the age of 6 weeks. Group 1 received a standard App vaccine (Porcilis APP, available from Intervet International BV, Boxmeer, The Netherlands), without polymyxin. This vaccine contains (per ml) 25 units of the repeats in toxins ApxI, II and III as well as a 42 kD outer membrane protein. LPS is present in an amount of about 5-6 x10⁵ IU/ml. Group 2 received the same vaccine but with added polymyxin B (as a sulphate salt), in the amount of 30 µg/ml (about 250 IU per ml). Group 3 received the same standard vaccine, but with added polymyxin E (as a sulphate salt), also in the amount of 30 µg/ml (about 250 IU per ml). The polymyxins were added at the final stage of vaccine formulation, i.e. when all other antigens were being mixed with the carrier Diluvac Forte (available from Intervet International BV, Boxmeer, The Netherlands).

Rectal temperatures were measured 20 hrs before vaccination, just before vaccination, and 2, 4, 7 and 24 hrs after vaccination. All animals per group were monitored for specific clinical symptoms (indicative of endotoxic shock) at 30 min and 1, 2, 4, 7 and 24 hours after vaccination. In particular the animals were checked for the following symptoms: less active, shivering, coughing, diarrhoea, vomiting, increased respiration rate, abdominal respiration, dyspnoea and death. On days 2-6 post-vaccination only a daily check was performed. Temperature values and clinical symptoms of groups 2 and 3 were compared with those of group 1.

In a second study, two different concentrations of polymyxin were tested. This study was performed with 42 pigs, which were randomly assigned to 3 treatment groups of 14 pigs each. Group 1 received Porcilis APP (without polymyxin), group 2 the same vaccine with 30 µg polymyxin B per ml vaccine and group 3 the same vaccine with 60 µg polymyxin B per ml vaccine. Further experimental set-up was the same as in the first study.

In a third study the efficacy of an APP vaccine, based on LPS and containing polymyxin B was tested. Polymyxin B is commonly known as the polymyxin with the best LPS binding properties, and thus theoretically polymyxin B has the strongest negative impact on vaccine efficacy. So, if a vaccine formulated with this polymyxin is effective, this is proof that with another polymyxin at least an equally effective vaccine can be obtained. The vaccine for this study was formulated by adding 30 µg polymyxin B per ml vaccine to standard Porcilis APP vaccine. Fourteen pigs were vaccinated intramuscularly at the age of 6 and 10 weeks with 2 ml of the vaccine, thus receiving a dose of about 500 IU polymyxin. Seven pigs received a placebo vaccine (40 mM tris-HCl buffer). Antibody titers against the RTX toxins ApxI, ApxII and Apx III as well as OMP were determined at 13 weeks. Three weeks after the second vaccination, pigs were all challenged with *Actinobacillus pleuropneumoniae* serovar 2.

In a fourth study we tested the safety of a vaccine wherein the polymyxin is added during the down stream processing of an *Actinobacillus pleuropneumoniae* fermentation batch, ultimately arriving a far lower content of polymyxin in the vaccine (about 2-4 µg/ml). In this study the vaccine was prepared by adding 30 µg polymyxin B or polymyxin E sulphate per ml supernatant of the fermentation broth of the respective cultures, wherafter the toxins ApxI, II, III complexed with LPS, and the 42 kD OMP were isolated from these supernatants. These isolated subunits were formulated in Diluvac forte to arrive, per ml vaccine, at about 25 units for the toxins and OMP and about 1x10⁶ IU of LPS. In these vaccines a polymyxin concentration of about 22 IU (2.6 µg/ml) for polymyxin E and about 35 IU (4.2 µg/ml) for polymyxin B per ml was found. To test the safety, two groups of ten 6 weeks old SPF pigs each received 4 ml of the respective vaccine. Clinical symptoms were assessed as in the first study.

### Results

There was no significant effect of either of the polymyxins on rectal temperatures. However, there were significant effects with regard to symptoms of endotoxic shock. In Table 1 here-beneath a summary of the symptoms per group is given. In group 1 typical clinical symptoms were observed in more than half of the animals, like vomiting (60%) and increased respiration rate (66%). In this group two pigs (4%) died as a result of endotoxic shock. In contrast, only very mild symptoms were observed in groups 2 and 3. In both groups 2% of the pigs (one out of 50 animals) were vomiting. In addition, 2% of the pigs of group 3 had an increased respiration rate. It is therefore concluded that polymyxins are suitable for reducing severe endotoxic shock symptoms caused by the LPS in the APP vaccine.

**Table 1. Symptoms of endotoxic shock after vaccination with various APP vaccines**

| Vaccine | diarrhoea | vomiting | increased respiration rate | death |
|---|---|---|---|---|
| Porcilis APP | 10% | 60% | 66% | 4% |
| Porcillis APP + 30 µg/ml polymyxin B | 0% | 2% | 0% | 0% |
| Porcilis APP + 30 µg/ml polymyxin E | 0% | 2% | 2% | 0% |

Table 2 shows the results of the second study. In group 1 (standard vaccine) typical clinical symptoms like vomiting (54%) and an increased respiration rate (15%) were observed. In groups 2 and 3 mild to nearly no symptoms were observed. Based on these results, it is concluded that adding as little as 60 µg of a polymyxin per ml vaccine, or even as little as 30µg/ml is effective to almost completely block the LPS in inducing severe symptoms of endotoxic shock.

**Table 2. Symptoms of endotoxic shock after vaccination with various APP vaccines**

| Vaccine | diarrhoea | vomiting | increased respiration rate | death |
|---|---|---|---|---|
| Porcilis APP | 15% | 54% | 15% | 0% |
| Porcillis APP + 30 µg/ml polymyxin B | 0% | 0% | 8% | 0% |
| Porcilis APP + 60 µg/ml polymyxin B | 0% | 0% | 0% | 0% |

In the third study, good efficacy results (equal to results obtainable with standard Porcilis APP vaccine having the same formulation except for the polymyxin) were obtained with the vaccine containing polymyxin B. The results are summarised in table 3. In the control group nearly 60% of the animals died, whereas the animals in the vaccinated group were all protected against challenge. Titers in the latter group were comparable with those obtainable with standard Porcilis APP vaccine not containing a polymyxin ("n.d." means not detectable in the test used). Given the extremely good results with an APP vaccine containing polymyxin B, and the fact that this polymyxin has the strongest LPS binding properties, it is understood that with other polymyxins a vaccine can be obtained that is at least equally effective.

**Table 3. Average efficacy results of a vaccine containing polymyxin B**

| Vaccine | Apx I titer | ApxII titer | Apx III titer | OMP titer | death |
|---|---|---|---|---|---|
| Porcilis APP + 30 µg/ml polymyxin B | 9.3 | 10.1 | 8.5 | 9.1 | 0/14 |
| control | n.d. | n.d. | n.d. | n.d. | 4/7 |

In the fourth study it was shown that even with a vaccine comprising as little as about 20 IU polymyxin per ml, good safety results can be obtained. The mean data are depicted in Table 4. Given the effect that even with a significantly higher polymyxin content (see study 3) still good efficacy results can be obtained, it is clear that with the vaccines of the fourth study good efficacy results can be obtained as well.

**Table 4. Symptoms of endotoxic shock after vaccination with various APP vaccines**

| Vaccine | diarrhoea | vomiting | increased respiration rate | death |
|---|---|---|---|---|
| Porcilis APP | 50% | 90% | 70% | 0% |
| Porcillis APP ctg 4.2 µg/ml polymyxin B | 0% | 10% | 10% | 0% |
| Porcilis APP ctg 2.6 µg/ml polymyxin E | 0% | 20% | 30% | 0% |

## Claims

1. A vaccine directed against porcine pleuropneumonia, comprising lipopolysaccharide, **characterised in that** the vaccine comprises a polymyxin to reduce symptoms of an endotoxic shock arising from the lipopolysaccharide.

2. A vaccine according to claim 1, **characterised in that** the vaccine comprises lipopolysaccharide purified from a bacterial culture complexed with one or more repeats in toxins.

3. A vaccine according to claim 2, **characterised in that** it the repeats in toxins are ApxI, Apxll and Apxlll.

4. A vaccine according to any of the claims 1 to 3, **characterised in that** the vaccine comprises less than 2000 IU of polymyxin per dosis.

5. A vaccine according to claim 4, **characterised in that** the vaccine comprises less than 1000 IU of polymyxin per dosis.

6. A vaccine according to claim 5, **characterised in that** the vaccine comprises less than 500 IU of polymyxin per dosis.

7. A vaccine according to any of the preceding claims, **characterised in that** the polymyxin is polymyxin B.

8. A vaccine according to any of the preceding claims, **characterised in that** the lipopolysaccharide originates from *Actinobacillus pleuropneumoniae.*

9. A vaccine according to any of the preceding claims, **characterised in that** the vaccine comprises a 42 kD outer membrane protein of *Actinobacillus pleuropneumoniae.*

10. A method to obtain a vaccine directed against porcine pleuropneumonia, comprising obtaining lipopolysaccharide, mixing the lipopolysaccharide with a pharmaceutically acceptable carrier and detoxifying the lipopolysaccharide by adding a polymyxin.

11. A method according to claim 10, **characterised in that** polymyxin is added to arrive at a maximum of 2000 IU per dosis vaccine.

12. A method according to claim 11, **characterised in that** polymyxin is added to arrive at a maximum of 1000 IU per dosis vaccine.

13. A method according to claim 12, **characterised in that** polymyxin is added to arrive at a maximum of 500 IU per dosis vaccine.

14. A method according to any of the claims 10 to 13, **characterised in that** the polymyxin is added when the lipopolysaccharide is mixed with the carrier.

15. Use of a polymyxin to reduce symptoms of an endotoxic shock when administering a vaccine directed against porcine pleuropneumonia, **characterised in that** the polymyxin is added to the vaccine before administration to a subject animal.

16. A method to reduce symptoms of an endotoxic shock when administering a vaccine directed against porcine pleuropneumonia containing lipopolysaccharide, comprising administering a vaccine according to any of the claims 1 to 9.
